# EUROPEAN PATENT APPLICATION

(11) **EP 4 830 678 A1**
(43) Date of publication of application: **28.10.2026**
(21) Application number: 25201750.4
(22) Date of filing: 11.09.2025
(51) Int. Cl.: A61F 13/49, A61F 13/496

(54) **ABSORBENT ARTICLE WITH IMPROVED WAIST**

(30) Priority: 23.04.2025 EP 25172049; 16.05.2025 EP 25176943; 17.06.2025 EP 25183175
(71) Applicant: Ontex BV, 9255 Buggenhout (BE); Ontex Group NV, 9320 Erembodegem (BE)
(72) Inventor: BUYSSE, Michiel, 9968 Oosteeklo (BE); PSCHYKLENK, Lukas, 56761 Müllenbach (DE); FRANSEN, Markus, 56269 Dierdorf (DE)
(74) Representative: Macchetta, Andrea

(57) **Abstract**

An absorbent article comprising: a front waist belt; a rear waist belt; an absorbent insert joined to the front and rear waist bets; wherein the front and rear waist belts each comprise an outer nonwoven layer, an inner nonwoven layer, and an elastic material sandwiched between said outer and inner nonwoven layers; the front and rear waist belts being joined together at a pair of side seams to form a waist opening; and wherein at least one, preferably both, of the front and rear waist belts comprise a cushioning composite at the waist opening.

## Description

### TECHNICAL FIELD

The present disclosure pertains to the technical field of absorbent hygiene articles such as disposable diapers or (diaper) pants, disposable incontinence diapers or pants. Preferably the disclosure relates to disposable pants, more preferably disposable baby pants.

### BACKGROUND

Absorbent articles for personal hygiene are designed to absorb and contain bodily exudates, such as a large quantity of urine and/or stool. Disposable absorbent pants, such as baby diapers or adult incontinence garments, are designed to be worn for extended periods. Prolonged contact with the skin can lead to discomfort, skin irritation, or pressure marks, particularly for articles that are elasticised to guarantee optimal fit with movement. To improve wearer comfort and minimize the risk of skin rashes or marks, there is a continued need for disposable pants that are soft to the touch and gentle on the skin, especially in areas where the product contacts the body closely and which is prone to material or skin folding, such as the waist region.

The present disclosure aims to provide a novel absorbent article particularly designed to even more reduce the risks of pressure marks and the like. Other objects and advantages of this disclosure will become apparent hereinafter.

### SUMMARY

The disclosure relates to an absorbent article (1), preferably in the form of a pant, comprising: a front waist belt (2); a rear waist belt (3); an absorbent insert (4) joined to the front and rear waist bets (2, 3); wherein the front and rear waist belts (2, 3) each comprise an outer nonwoven layer (5), an inner nonwoven layer (6), and an elastic material (7) sandwiched between said outer and inner nonwoven layers (5, 6); the front and rear waist belts being joined together at a pair of side seams (8, 8') to form a waist opening (Ow); and wherein at least one, preferably both, of the front and rear waist belts (2, 3) comprise a cushioning composite (9) at the waist opening (Ow).

### BRIEF DESCRIPTION OF THE FIGURES

FIG. 1 is a schematized top plan view of an absorbent article according to embodiments herein, in a flat-out, uncontracted state (i.e., without elastic induced contraction) with the portion of the article which contacts the wearer facing the viewer.
FIG. 2 is a schematized perspective view of the article of FIG. 1, according to an embodiment herein.
FIG. 3 is a schematized section view of a portion of a waist belt of the article of Fig.1, according to an embodiment herein.
Fig. 4 is a schematized section view of a portion of a waist belt of the article of Fig.1, according to an embodiment herein.
FIG. 5 is a schematized top plan view of a topsheet comprising zone treatment according to embodiments herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural unless the context clearly dictates otherwise. By way of example, "a compartment" refers to one or more compartments.

"About" as used herein referring to a measurable value such as a parameter, an amount, a temporal duration, and the like, encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants and diaper holders and liners, Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles and the components thereof can operate to provide a skin-facing surface and a garment-facing surface.

The "absorbent core" is the absorbent structure disposed between the topsheet and the backsheet of the chassis and is capable of absorbing and retaining liquid body exudates. The size and the absorbent capacity of the absorbent core should be compatible with the size of the intended wearer and the liquid loading imparted by the intended use of the chassis. Further, the size and the absorbent capacity of the absorbent core can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent core. It can contain embossed channels, channels substantially free of material, channels with lower basis weight than the rest of the core, etc. The absorbent core is disposed between the topsheet and the backsheet; both the topsheet and the backsheet generally extend beyond the side edges of the absorbent core. The juxtaposed areas of the topsheet and the backsheet are typically secured together such as by adhesive.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help to prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface in the required pattern or network of adhesive areas. Accordingly, suitable adhesives include conventional hot melt adhesives, pressure-sensitive adhesives and reactive adhesives (i.e., polyurethane).

"Bonded" refers to the joining, adhering, connecting, attaching, or the like, of at least two elements. Two elements will be considered to be bonded together when they are bonded directly to one another or indirectly to one another, such as when each is directly bonded to intermediate elements. "Join", "joining", "joined", or variations thereof and "attach", "attaching", "attached", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined or attached together either directly or indirectly, i.e. they may have one or more elements interposed between them, all of which are connected together. For the sake of clarity, adhesive is not to be considered as an intervening element for the definition of direct or indirect attachment. When describing an attachment "without intervening layer of material" or "without any intervening material", this does not exclude the presence of adhesive.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "associated" encompasses configurations in which two elements are directly or indirectly joined. For example configurations where top sheet is directly joined to back sheet by affixing top sheet directly to back sheet, and configurations wherein top sheet is joined to back sheet by affixing top sheet to intermediate members which in turn are affixed to back sheet. Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The term "backsheet" refers to a material forming a liquid impervious layer. The back sheet generally prevents the exudates contained in the absorbent core from wetting articles such as bedsheets and overgarments which contact the disposable absorbent article. The backsheet may be a composite layer composed of multiple components assembled side-by-side or laminated. The backsheet may be the same or different in different parts of a chassis. The backsheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials, in such laminates, the polymeric film can have the same or a different width than the nonwoven material. The term "breathable" refers to films having a water vapor transmission rate (WVTR) of at least 300 grams/m² - 24 hours.

As used herein, the "skin-facing" or "bodyside" or " body-facing" or "user facing" or "inner" side or surface means that surface of the article or component which is intended to be disposed toward or placed adjacent to the body of the wearer during ordinary use, while the "outward", "outward-facing", "garment-facing" or "garment" or "outer" side or surface is on the opposite side, and is intended to be disposed to face away from the wearer's body during ordinary use. Such outward surface may be arranged to face toward or placed adjacent to the wearer's undergarments when the absorbent article is worn.

Further, an absorbent article can comprise "standing gathers" or "containment flaps" or "barrier cuffs". The standing gathers are generally thought to be particularly well suited for the containment of fecal matter and to prevent the lateral flow of liquid waste until such time as the liquid waste can be absorbed by the absorbent article. Many constructions of standing gather are known. Such standing gather generally comprises a proximal portion intended to be attached to the chassis, and an opposed distal portion which is generally not attached to the chassis along at least a portion of its length. An elastic member is generally located on or adjacent a distal edge to provide elasticity to the standing gather, to assist in maintaining the containment flap in an upright condition and in maintaining a sealing relationship between the distal edge of the standing gather and the body of a wearer during use. The elastic member is generally located between two layers of material so that the elastic does not come into contact with the body of a wearer. The standing gather may be manufactured from any of a wide variety of materials such as woven, nonwoven, spunbonded, carded, cast, blown or the like, or combinations or laminations thereof. The materials may include any of a variety of compositions including but not limited to polypropylene, polyester, rayon, nylon, foams, plastic films, formed films, and elastic foams. To improve containment, the material may be hydrophobic, or at least partially liquid impervious. A number of manufacturing techniques may be used to manufacture the containment flaps.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

As used herein, the term "impermeable" or "impervious" generally refers to articles, chassis and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

The term "flat configuration" or "laid-flat state" or "fully stretched state" or "extended state" or "flat-out" configuration is intended to refer to the article when it is flattened into a plane or is substantially flattened into a plane and is used in contrast to when the article otherwise positioned, such as when the article is folded or shaped in or for use by a wearer.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "topsheet" refers to a liquid permeable material sheet typically forming the inner cover of of an absorbent article and which in use is placed in direct contact with the skin of the wearer. The topsheet is typically employed to help isolate the wearer's skin from liquids held in the absorbent structure. The top sheet can comprise a nonwoven material, e.g., spunbond, meltblown, carded, hydroentangled, wetlaid or any other type of nonwoven. Suitable nonwoven materials can be composed of man-made fibres, such as polyester, polyethylene, polypropylene, viscose, rayon etc. or natural fibers, such as wood pulp or cotton fibres, hemp, and the like, or other natural or naturally-derived fibers, or from a mixture of two or more natural or man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g., urine or menstrual fluid. The inner coversheet may further be different in different parts of the chassis of an absorbent article. The top sheet fabrics may be composed of a substantially hydrophobic material, and the hydrophobic material may optionally be treated with a surfactant or otherwise processed to impart a desired level of wettability and hydrophilicity.

As used herein, an "air-through-bonded" nonwoven, is a nonwoven structure primarily formed by a process that comprises the application of heated air to the surface of the nonwoven fabric. During the through air bonding process, heated air flows through holes in a plenum above the nonwoven material. Unlike hot ovens, which push air through the material, the through air process uses negative pressure of suction to pull the air through, for example, an open conveyor apron holding nonwoven as it is drawn through the oven. Pulling air through the material allows the rapid and even transmission of heat to minimize distortion of the nonwoven material. The binding agents used in the through air bonding process include crystalline binder fibers and powders, which melt to form molten droplets throughout the cross-section of the nonwoven. As the material is cooled, bonding occurs at these droplet points.

As used herein, the term "transverse" or "lateral" refers to a line, axis, or direction which lies within the plane of the absorbent article and is generally perpendicular to the longitudinal direction (i.e., is perpendicular to the y axis).

The term "bio-based" may be used herein to refer to a component of an absorbent article that can be produced or is derived from a renewable resource. The term "renewable resource" is used herein to refer to a natural resource that may be produced by a natural process at a rate comparable to its rate of consumption (e.g., within a 100 year time frame). The resource may be replenished naturally, or via engineered agricultural techniques. Nonlimiting examples of renewable resources may include plants, animals, fish, bacteria, fungi, and forestry products. These resources may be naturally occurring, hybrids, or genetically engineered organisms. Natural resources such as crude oil, coal, and peat that take longer than 100 years to form are generally not considered to be renewable resources.

"Natural fibers" refers to elongated substances produced by plants and animals and includes animal-based fibers and plant-based fibers, as those categories are described herein. Natural fibers, as that term is used herein, include fibers harvested without any post-harvest treatment step, as well as those having a post-treatment step, such as, for example, washing, scouring, bleaching, etc.

"Plant-based fibers," as used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut.

"Cushioning composite", as used herein generally refers to a component of the absorbent article, preferably being part of a waist belt thereof (e.g. a front and/or back waist belt), that typically provides some cushioning, for example by reducing or alleviating pressure onto the skin of a wearer.

Embodiments according to the disclosure will now be described. It is understood that technical features described in one or more embodiments may be combined with one or more other embodiments without departing from the intention of the disclosure and without generalization therefrom.

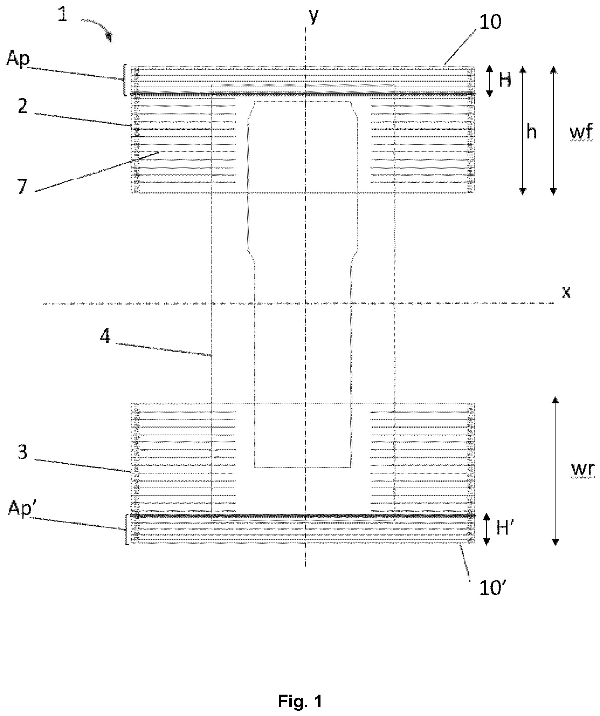

Absorbent articles (1), according to the present disclosure, as for example illustrated in Fig.1-4, are preferably in the form of a pant. The articles herein comprise: a front waist belt (2); a rear waist belt (3); an absorbent insert (4) joined to the front and rear waist bets (2, 3); wherein the front and rear waist belts (2, 3) each comprise an outer nonwoven layer (5), an inner nonwoven layer (6), and an elastic material (7) sandwiched between, preferably directly between (i.e. generally meaning without further intervening layers except for the joining means such as adhesive), said outer and inner nonwoven layers (5, 6); the front and rear waist belts being joined together at a pair of side seams (8, 8') to form a waist opening (Ow). At least one, preferably both, of the front and rear waist belts (2, 3) comprise a cushioning composite (9) at the waist opening (Ow). Advantageously, it has been found that introduction of a cushioning composite at the waist opening improves comfort and reduces the formation of skin sores by in essence generally increasing the total surface area of contact which in turn results in reduced pressure at a given applied force (e.g. a contraction force generated by the elastic material within the belts to provide for optimal fit). Such reduced effective pressure is beneficial to reduce pressure marks on the skin of a wearer.

Preferably, the cushioning composite (9) herein is dimensioned such to increase a contact area to a skin surface of a wearer compared to an otherwise identical waist belt being free of said cushioning composite (9).

Absorbent inserts (4) for use herein preferably comprise a liquid-pervious topsheet, typically defining at least a portion of the user-facing or skin-facing side of the insert (or sometimes herein referred to as chassis), a backsheet, typically defining at least a portion of the garment-facing side of the insert, said backsheet preferably being associated or joined to the topsheet, and am absorbent core disposed between the liquid-pervious topsheet and the backsheet. The backsheet may comprise a liquid impervious film layer and preferably an outer cover layer. The insert (4), as for example illustrated in Fig. 1, has a front region, a back region, and an intermediate crotch region, which interconnects the front and back regions, and a periphery which is defined by the outer edges of the chassis in which first and second longitudinal side edges connect to front and back transverse edges. The insert may additionally have a lateral axis x which is here drawn as a centerline and a longitudinal axis y which is here drawn as a centerline. The longitudinal axis y generally extends through the front and back waist belts, and the lateral axis x is substantially perpendicular to the longitudinal axis. While the insert (4) is illustrated in FIG. 1 as having a generally rectangular shape, it may have other shapes or contours, e.g. an hourglass shape, as desired.

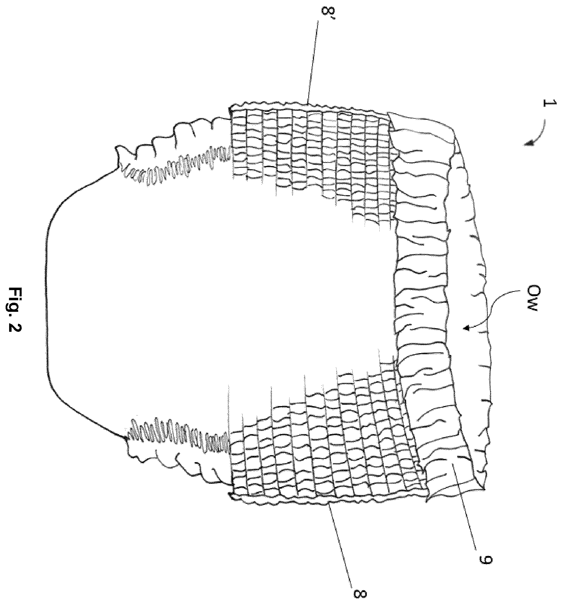

For example, as illustrated in Fig. 1 and 2, the article is in the form of a pant and may comprise front and back waist belts (or panels) 2, 3. These belts/panels are generally adapted to come into contact with the waist of the user and are preferably joined to each other through at least two opposite side seams 8, 8' that may be permanent or refastenable. The belts/panels preferably comprising a plurality of first elastics and/or one or more second elastics, preferably wherein the elastic spacing between first elastics is different, more preferably greater than, the elastic spacing between second elastics.

The articles herein may further comprise one or more transversal cuffs and/or pockets at the front and/or back of said article. Such pockets may be as for example described on paragraphs 0072 and 0073 of EP4454624A1 to which cross reference is made herein.

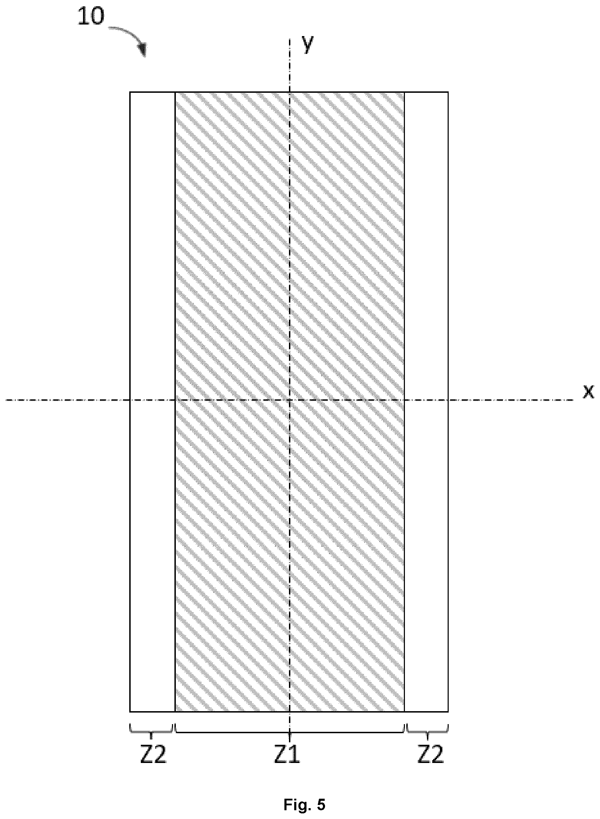

As exemplified in Fig. 5, the topsheet herein may comprise one or more first zones (Z1) and at least two second zones (Z2) wherein the first zone(s) (Z1) is substantially hydrophilic [typically meaning that at least semi-durable hydrophilic properties are imparted as explained in more detail in embodiments herein] and wherein the second zones (Z2) are hydrophobic; and in that an attached portion of each of a pair of barrier cuffs overlaps at least one of the second zones (Z2) and does not substantially overlap [typically meaning that minor overlaps, e.g. due to production tolerances, are encompassed], preferably does not overlap, the first zone(s) (Z1); and/or in that the attached portion of each of said barrier cuffs is positioned proximal or adjacent to a second zone (Z2) and distal from said first zone(s) (Z1) such that the attached portion is separated from said first zone(s) (Z1) by said second zone (Z2) preferably in a width direction extending substantially parallel to the lateral axis x. Advantageously this arrangement allows to significantly reduce the risk of leakage that could otherwise arise from liquid permeating through the hydrophilic portions of the topsheet under the cuffs.

Nonwovens made from synthetic fibers, such as polyolefins like polypropylene, polyethylene and the like, are generally hydrophobic in nature. By application of suitable finishing treatments (also referred to as spinfinish), it is possible to impart semi-durable hydrophilic properties to the nonwoven to achieve performance in liquid strike-through and liquid runoff that is required for their use in absorbent products. Suitable finishing treatments are typically proprietary blends of synthetic surfactant solutions which are commercially available, for example, from Schill & Seilacher AG (e.g. Silastol PHP 26, Silastol PHP 90, & Silastol 163), and Pulcra Chemicals (e.g. Stantex S 6327, Stantex S 6087-4, & Stantex PP 602). Traditionally they are typically applied to the nonwovens full surface in the range of 0.004-0.006 gm solids/gm nonwoven (i.e. 0.4-0.6% wt/wt). An example of a synthetic surfactant that has been widely used in commercially-available topsheet finishes would be Triton GR-5M, an anionic sulfosuccinate surfactant manufactured by Dow Chemical Company. Other types of surfactants used are based on fatty acid polyethylene glycol esters. Any such spinfinish solutions, and the like, are suitable herein as long as applied according to the zones (or zone treatment) described herein.

The second zone(s) (Z1) is preferably substantially interposed between at least two of the said second zones (Z2), said at least two of the said second zones (Z2) extending longitudinally along the first and second longitudinal edges of the insert (4). The hydrophobic zones arranged in this manner may form retarding barriers to liquid.

It is preferred that said article is a pant, preferably of the disposable kind, comprising discrete front and back waist belts (also referred to "panels") (2, 3) wherein the absorbent insert (4) is joined to each of said front and back waist belts so that said belts are indirectly connected to each other by said insert, and preferably directly connected to each other at a pair of side seams (8, 8') to form a waist opening and two leg openings.

Preferably, the first zone(s) (Z1) is located centrally of the topsheet and the second zones are located outboard of the first zone(s) (Z1) and are positioned along at least a portion of a perimeter of the topsheet preferably at least comprising longitudinal edges running substantially parallel to the first and second longitudinal side edges of the insert (4). Advantageously this arrangement enhances the formation of retarding barriers to liquid and work synergistically with the cuffs as arranged as described herein.

The topsheet may comprise, preferably consists of, synthetic fibers that are generally hydrophobic, and wherein the first zone(s) (Z1) is hydrophilically treated with a substance, said substance preferably comprising one or more surfactants.

A suitable topsheet may be manufactured from a wide range of materials, such as apertured plastic films, woven materials, nonwoven materials, woven or nonwoven materials of natural fibers (e.g., wood or cotton fibers), synthetic fibers or filaments (e.g., a polyester or a polyolefin, such as polyethylene (PE), polypropylene (PP), or bicomponent PE/PP fibers or mixtures thereof), or a combination of natural and synthetic fibers. According to the present disclosure, the topsheet 10 may be a nonwoven material having one or multiple formed layers, nonwovens, or components comprising plant-based fibers and/or fibers comprising a recycled content of for example more than 50%, preferably other than wood pulp. The nonwoven may be made through well-known techniques; for example, the nonwoven may be a spunbond, a carded and air-through, hydroentangled, or calendar bonded nonwoven. The plant-based fibers may also be spun fibers made at least in part from bio-based materials. For example, the plant-based fibers may be spun poly lactic acid (PLA) fibers or bio-based polyolefin spun fibers. The plant-based fibers may be single component fibers or multi-component fibers, in which less than all of the components are plant-based fibers.

One example is a bi-component fiber comprising a polyester core component and a bio-based polyethylene sheath component.

The topsheet may have one or more layers. The topsheet may be apertured, may have any suitable three-dimensional features, and/or may have a plurality of embossments. The topsheet may have hydrophilic and/or hydrophobic portions or layers. The topsheet 26 may have a basis weight of from about 10 gsm to about 40 gsm.

The present disclosure is particularly advantageous with, but not limited to, topsheet materials which may have at least one of the following characteristics: bulky, lofty, soft, with visual embossing, with perforations, air-through bonded, spunbond, spunmelt, calendered, highloft, semi-highloft, and/or which may comprise micro-fibers, PE, PET, and/or bio-based polymers as for example PLA. In some embodiments, the topsheet is selected from air-through bonded nonwovens, embossed nonwovens and nonwovens comprising plant-based fibers. An exemplary topsheet is a spunbond nonwoven from PFN, 100 Wt% PP. In some embodiments, the liquid-pervious topsheet 10 consists of a single nonwoven layer or consists of a nonwoven underlayer on which an upper nonwoven patch is attached, said patch being shorter than the nonwoven underlayer in both width along the lateral axis X-X and length along the longitudinal axis Y-Y. The use of such a patch may provide loftiness, bulkiness, premium sensation and/or embossing in a dedicated zone where it is most wanted, while saving on material.

The insert (4) may further include an acquisition and distribution layer between the core and the topsheet and/or between the core and the backsheet.

The elastic material for use herein is generally selected from the group consisting of an elastic film, elastic strands, and combinations thereof. Preferred is the use of elastic strands as exemplified for example in Fig.1. Advantageously, strands allow for an improved breathability of the laminate compared to film (even when perforated) as well as reduced waste upon disposal.

The cushioning composite (9) herein may comprise an air gap (G) and one or more nonwoven layers, preferably at an apex position/region (Ap, AP') of the front and/or rear waist belts (2, 3) that circumscribes the opening (Ow). Advantageously, such air gap allows to further increase the surface area of contact with the skin of the user by allowing the nonwoven layers to not stick to each other and effectively increase the surface area that is available for contact compared to for example nonwoven layers that are tightly pressed together or in contact for example by use of adhesive (or elastics that are strand-coated with adhesive).

Preferably, the cushioning composite (9) is free of an elastic material. Advantageously this allows the cushioning composite to provide increased softness perception.

The cushioning composite (9) herein may be formed by one or both of the inner and outer nonwoven layers (6, 5). Advantageously this allows for reduced material waist and/or manufacturing complexity. Indeed, whilst the cushioning composite (9) may be formed by a third (distinct) nonwoven that is joined to the inner and/or outer nonwoven layers, such further material likely would result in less desirable addition of material (e.g. at least more adhesive to affect the joining of such a further distinct element) and more complex processing steps for attaining such joining that may be less desirable when aiming at high processing speeds of more than 800 pieces per minute, and hence is less preferred.

The outer nonwoven layer (5) is generally wider (such width dimension preferably being taken along the y axis as illustrated and explained in further detail herein below) than the inner nonwoven layer (6) and is folded over the inner nonwoven layer (5) at a waist edge (10, 10') and wherein an air gap (G) is formed between the outer and inner nonwoven layers (6, 5) at said waist edge (10, 10'). As explained above, the Air gap (G) herein is generally formed by ensuring nonwoven layers such as inner and outer nonwoven layers are not associated or joined to each other and preferably the nonwoven layers (such as inner and outer nonwoven layers) being differently sized so that separation of said layers in the form of a substantially bubble-shape is possible. Advantageously a cost-effective and environmentally friendly cushioning can be achieved.

An apex position (Ap, Ap') of the front and/or rear waist belts (2, 3) that circumscribes the opening (Ow) may comprise the waist edge (10, 10'), preferably wherein the waist edge (10, 10') corresponds to an apex of the front and/or rear waist belts (2, 3) that circumscribes the opening (Ow). Advantageously, in order to limit cost and material, it has been found that targeting the particular upper waist portion of the waist belt(s) to include such cushioning achieves the desired softness on the skin.

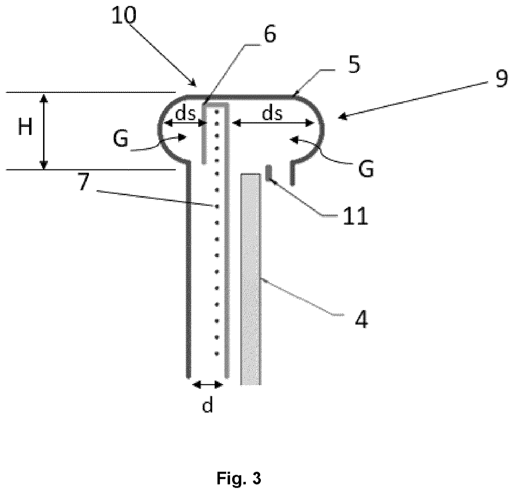

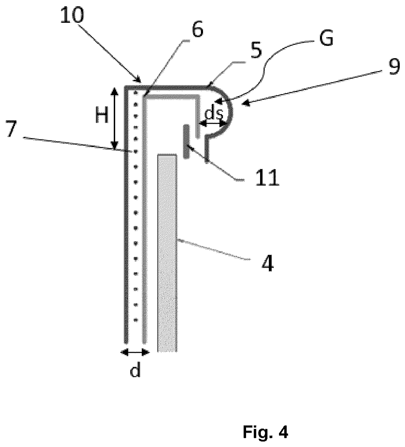

As exemplified in Fig. 3 and Fig. 4, the cushioning composite (9) herein preferably comprises no more than two, more preferably two, nonwoven layers separated from each other by an air gap (G) such to form a substantially bubble-shaped waist rim, and preferably wherein the two nonwoven layers are the inner and outer nonwoven layers herein. Advantageously, such bubble-shaped rim aids to provide a further perception of softness to the wearer and the care giver handling the article.

Preferably, a separation distance (ds) separates the two nonwoven layers at the bubble-shaped waist rim, and the outer nonwoven layer (5) and inner nonwoven layer (6) are separated from each other at one or more portions other than the substantially bubble-shaped waist rim by a distance (d), and wherein the separation distance (ds) is greater than the distance (d), preferably wherein the separation distance (ds) is at least 1.2d, even more preferably the separation distance (ds) is from 1.5d to 20d. Preferably, the separation distance is from 1.6d to 15d, even more preferably from 1.8d to 12d, even more preferably from 2d to 10d. Advantageously such separation allows to form a suitable bubble-shaped rim for extra softness.

The cushioning composite is preferably positioned on a skin-facing surface of the front and/or rear belts (2, 3) [as for example illustrated in Fig. 4] and/or is positioned on the garment-facing surface of the front and/or rear belts (2, 3); and preferably is positioned on both the skin-facing surface and the garment-facing surface of the front and/or rear belts (2, 3) [as for example illustrated in Fig.3].

The inner and outer nonwoven layers (6, 5) may be different in at least one of basis weight, fiber composition, nonwoven type, and combinations thereof. Such modular difference may be advantageous to drive further softness where it is needed whilst limiting the cost and/or material usage.

Preferably, one of the inner nonwoven layer (6) or outer nonwoven layer (5) has a basis weight that is greater than the other of the outer nonwoven layer (5) or the inner nonwoven layer (6), preferably wherein the outer nonwoven layer (5) has a basis weight that is greater than that of the inner nonwoven layer (6), even more preferably wherein the outer nonwoven layer (5) has a basis weight that is at least 5gsm greater than the inner nonwoven layer (6). Preferably the outer nonwoven layer (5) has a basis weight that is at least 8gsm greater, even more preferably at least 10gsm greater, even more preferably at least 12gsm greater, even more preferably at least 15gsm greater, than the inner nonwoven layer (6). The inner nonwoven layer (6) herein generally having a basis weight of from 8gsm to 25gsm, preferably from 10gsm to 20gsm. Counterintuitively. such difference may be advantageous to drive further softness where it is needed whilst limiting the cost and/or material usage.

The inner nonwoven layer (6) herein may be folded such to comprise an outer fold (as exemplarily shown in Fig. 3) having a length that substantially corresponds to the height (H, H') of the cushioning composite (9) preferably wherein at least one, preferably a plurality of, elastic strand(s) is sandwiched between adjacent surfaces of the folded nonwoven (6). Preferably an air gap (G) being comprised between an outer surface of the folded inner nonwoven (6) and a skin-facing surface of the outer nonwoven layer (5). In this preferred embodiment, a further air gap (G) may be comprised between a skin-facing surface of the inner nonwoven layer (6) and a folded portion of the outer nonwoven layer (5) positioned substantially opposite the outer fold of the inner nonwoven layer (6). An end position of the folded portion of the outer nonwoven layer (5) being generally joined to the skin-facing surface of the inner nonwoven layer (6) and/or insert (4) at a bond location (11). The joining a the bond location may be done by any suitable means such as adhesive and/or mechanical bonding such as heat pressure bonding, ultrasonic bonding and the like. Advantageously an effective cushioning composite is attained with minimal added materials and process/manufacturing complexity and this whilst being able to generate inner and outer bubble-shaped cushioning.

In addition or alternatively to the above, the inner nonwoven layer (6) herein may be folded such to comprise an inner fold (as exemplarily shown in Fig. 4) having a length that substantially corresponds to the height (H, H') of the cushioning composite (9) preferably wherein no elastic strand(s) are sandwiched between adjacent surfaces of the folded nonwoven (6). Preferably an air gap (G) being comprised between an inner surface of the folded inner nonwoven (6) and a garment-facing surface of the outer nonwoven layer (5). In this other preferred embodiment, the outer nonwoven layer (5) may be folded over the inner nonwoven layer (6) in the same direction of the inner fold of the inner nonwoven layer (6) so that a folded portion of said outer nonwoven layer (5) extends along the length of the inner fold (generally substantially corresponding to the height **H,** H'). An end position of the folded portion of the outer nonwoven layer (5) being generally joined to the skin-facing surface of the inner nonwoven layer (6) and/or insert (4) at a bond location (11). The joining a the bond location may be done by any suitable means such as adhesive and/or mechanical bonding such as heat pressure bonding, ultrasonic bonding and the like. Advantageously an effective cushioning composite is attained with minimal added materials and process/manufacturing complexity.

The outer nonwoven layer (5) generally comprises, preferably consists of, a nonwoven selected from the group consisting of a spunbond nonwoven layer, a carded nonwoven layer, and combinations thereof, more preferably wherein the outer nonwoven layer is a carded air-through-bonded nonwoven layer or a spunbond nonwoven layer (where the spunbond nonwoven layer is preferably a spunbond through-air-bonded nonwoven). Advantageously this has been found to provide for additional softness in the arrangements herein.

The outer nonwoven layer (5) preferably has a density of between 20 and 400 kg/m3, preferably between 20 and 300 kg/m3, more preferably between 20 and 250 kg/m3, even more preferably between 20 and 200 kg/m3, even more preferably from 25 to 120 kg/m3. Advantageously, by providing an outer nonwoven layer (5) with a relatively low density (i.e. a relatively open structure) the nonwoven may provide additional softness.

The density may vary across the thickness of the outer nonwoven layer (5). Preferably, the density is lower at the outermost surface of the outer nonwoven layer (5) (i.e. the surface thereof closest to a garment of a wearer and furthest from the skin of a wearer). Advantageously this allows to retain softness to the touch yet maintain good structural integrity for lamination with elastics that are generally applied on the skin-facing surface of the outer nonwoven layer and hence the higher density region thereof.

The inner nonwoven layer (6) preferably has a second density of between 100 and 400 kg/m3, said second density being higher than the density of the outer nonwoven layer (6), wherein a difference between the density and the second density is higher than 20 kg/m3, preferably higher than 30 kg/m3, more preferably higher than 40 kg/m3, even more preferably of between 50 and 150 kg/m3, most preferably of from 65 and 135 kg/m3. Advantageously a good balance between softness and structural integrity of the laminate may be achieved.

For determining the density of the nonwovens herein, a mass per unit area may be measured using NWSP 130.1. R0 (20) and a nonwoven thickness may be measured using NWSP 120.6. R0 (15). The density can then be calculated as the mass per unit area divided by the thickness.

The inner nonwoven layer (6) generally comprises at least one spunbond nonwoven layer and at least one meltblown nonwoven layer, preferably wherein the inner nonwoven layer (6) comprises, preferably consists of, a spunbond-meltblown-spunbond nonwoven layer. Advantageously, although such materials are less soft, they provide for added mechanical integrity and resistance to tear that allows for better supporting the elastic material that is sandwiched between the inner and/or outer nonwoven layers.

The cushioning composite (9) herein preferably has a height (H, H') extending substantially parallel to a longitudinal axis (y), and the front and rear waist belts (2, 3) have a front width (wf) and a rear width (wr) respectively each extending substantially parallel to the longitudinal axis (y), and wherein said height (H, H') is less than 50% of said front width (wf) and/or rear width (wr), preferably wherein said height (H. H') is from 5% to 30%, even more preferably from 8% to 20%, of said front width (wf) and/or rear width (wr). Advantageously this allows to attain softness perception where it is needed whilst minimizing impacts on fit for which an elasticized belt surface may be desirable.

The cushioning composite (9) herein preferably has a height (H) extending substantially parallel to a longitudinal axis (y), and wherein each one of the pair of side seams (8, 8') has a height (h) extending substantially parallel to a longitudinal axis (y), and wherein the height (H) of the cushioning composite (9) is less than 40% of the height (h) of the seam (8, 8'), preferably from 5% to 35%, even more preferably from 8% to 25%, of the height (h) of the seam (8. 8'). In embodiments where the rear waist belt (3) is wider than the front waist belt (2), the side seam height (h) corresponds to the front width (wf) and preferably such that a rear flange is formed having an unjoined height corresponding to **wr-wf.** Advantageously this allows to attain softness perception where it is needed whilst minimizing impacts on fit for which an elasticized belt surface may be desirable and yet further permit optimized buttocks coverage on the rear for better overall fit and resistance to leakage.

Preferably, the cushioning composite (9) herein is free of further nonwoven layers. This typically meaning that there are no further nonwoven layers beyond the inner nonwoven layer (6) and outer nonwoven layer (5) forming the cushioning component. Preferably the cushioning composite (9) is thus not a further component of the front and/or rear belts (2, 3) and is rather an integral part of the laminate forming the front and/or rear belts (2, 3). Advantageously this allows for the manufacturing/process decomplexification and reduction of material usage, waste etc.

Absorbent articles as herein described may take the form of a pant for babies, children or adults. They may include a re-usable outer shell that is coupled with insert 4, which acts as a disposable absorbent insert that is separable from an outer shell comprising front and back waist belts.

It is assumed that the present disclosure is not restricted to any form of embodiment described above and that some modifications can be made to the presented embodiments of the invention without departing from the scope of the embodiments as set forth in the claims that follow.

## Claims

1. An absorbent article (1), preferably in the form of a pant, comprising:
- a front waist belt (2);
- a rear waist belt (3);
- an absorbent insert (4) joined to the front and rear waist bets (2, 3);
wherein the front and rear waist belts (2, 3) each comprise an outer nonwoven layer (5), an inner nonwoven layer (6), and an elastic material (7) sandwiched between said outer and inner nonwoven layers (5, 6);
the front and rear waist belts being joined together at a pair of side seams (8, 8') to form a waist opening (Ow);
**characterised in that** the at least one, preferably both, of the front and rear waist belts (2, 3) comprise a cushioning composite (9) at the waist opening (Ow).

2. An absorbent article according to Claim 1 wherein the elastic material is selected from the group consisting of an elastic film, elastic strands, and combinations thereof.

3. An absorbent article according to any of the preceding Claims wherein the cushioning composite (9) comprises an air gap (G) and one or more nonwoven layers, preferably being positioned at an apex position (Ap, AP') of the front and/or rear waist belts (2, 3) that circumscribes the opening (Ow).

4. An absorbent article according to any of the preceding Claims wherein the cushioning composite (9) is formed by one or both of the inner and outer nonwoven layers (6, 5).

5. An absorbent article according to any of the preceding Claims wherein the outer nonwoven layer (5) is wider than the inner nonwoven layer (6) and is folded over the inner nonwoven layer (5) at a waist edge (10, 10') and wherein an air gap (G) is formed between the outer and inner nonwoven layers (6, 5) at said waist edge (10, 10').

6. An absorbent article according to Claim 5 wherein an apex position (Ap, Ap') of the front and/or rear waist belts (2, 3) that circumscribes the opening (Ow) comprises the waist edge (10, 10'), preferably wherein the waist edge (10, 10') corresponds to an apex of the front and/or rear waist belts (2, 3) that circumscribes the opening (Ow).

7. An absorbent article according to any of the preceding Claims wherein the cushioning composite (9) comprises two nonwoven layers separated from each other by an air gap (G) such to form a substantially bubble-shaped waist rim.

8. An absorbent article according to Claim 7 wherein a separation distance (ds) separates the two nonwoven layers at the bubble-shaped waist rim, and the outer nonwoven layer (5) and inner nonwoven layer (6) are separated from each other at one or more portions other than the substantially bubble-shaped waist rim by a distance (d), and wherein the separation distance (ds) is greater than the distance (d), preferably wherein the separation distance (ds) is at least 1.2d, even more preferably the separation distance (ds) is from 1.5d to 20d.

9. An absorbent article according to any of the preceding Claims wherein the inner and outer nonwoven layers (6, 5) are different in at least one of basis weight, fiber composition, nonwoven type, and combinations thereof.

10. An absorbent article according to Claim 9 wherein one of the inner nonwoven layer (6) or outer nonwoven layer (5) has a basis weight that is greater than the other of the outer nonwoven layer (5) or the inner nonwoven layer (6), preferably wherein the outer nonwoven layer (5) has a basis weight that is greater than that of the inner nonwoven layer (6), even more preferably wherein the outer nonwoven layer (5) has a basis weight that is at least 5gsm greater than the inner nonwoven layer (6).

11. An absorbent article according to any of Claims 9 to 10 wherein the outer nonwoven layer comprises, preferably consists of, a nonwoven selected from the group consisting of a spunbond nonwoven layer, a carded nonwoven layer, and combinations thereof, more preferably wherein the outer nonwoven layer is a carded air-through-bonded nonwoven layer or a spunbond nonwoven layer.

12. An absorbent article according to any of Claims 9 to 11 wherein the inner nonwoven layer (6) comprises at least one spunbond nonwoven layer and at least one meltblown nonwoven layer, preferably wherein the inner nonwoven layer (6) comprises, preferably consists of, a spunbond-meltblown-spunbond nonwoven layer.

13. An absorbent article according to any of the preceding Claims wherein the cushioning composite (9) has a height (H, H') extending substantially parallel to a longitudinal axis (y), and the front and rear waist belts (2, 3) have a front width (wf) and a rear width (wr) respectively each extending substantially parallel to the longitudinal axis (y), and wherein said height (H, H') is less than 50% of said front width (wf) and/or rear width (wr), preferably wherein said height (H. H') is from 5% to 30% of said front width (wf) and/or rear width (wr).

14. An absorbent article according to any of the preceding Claims wherein the cushioning composite (9) has a height (H) extending substantially parallel to a longitudinal axis (y), and wherein each one of the pair of side seams (8, 8') has a height (h) extending substantially parallel to a longitudinal axis (y), and wherein the height (H) of the cushioning composite (9) is less than 40% of the height (h) of the seam (8, 8'), preferably from 5% to 35% of the height (h) of the seam (8. 8').

15. An absorbent article according to any of the preceding Claims wherein the cushioning composite (9) is free of further nonwoven layers beyond the inner and outer nonwoven layers (6, 5).

## Amended claims

### Amended claims in accordance with Rule 137(2) EPC.

1. An absorbent article (1), preferably in the form of a pant, comprising:
- a front waist belt (2);
- a rear waist belt (3);
- an absorbent insert (4) joined to the front and rear waist bets (2, 3);
wherein the front and rear waist belts (2, 3) each comprise an outer nonwoven layer (5), an inner nonwoven layer (6), and an elastic material (7) sandwiched between said outer and inner nonwoven layers (5, 6);
the front and rear waist belts being joined together at a pair of side seams (8, 8') to form a waist opening (Ow);
**characterised in that** the at least one, preferably both, of the front and rear waist belts (2, 3) comprise a cushioning composite (9) at the waist opening (Ow) wherein the cushioning composite (9) comprises two nonwoven layers separated from each other by an air gap (G) such to form a substantially bubble-shaped waist rim; and **in that** the inner and outer nonwoven layers (6, 5) are different in at least one of basis weight, fiber composition, nonwoven type, and combinations thereof.

2. An absorbent article according to Claim 1 wherein the elastic material is selected from the group consisting of an elastic film, elastic strands, and combinations thereof.

3. An absorbent article according to any of the preceding Claims wherein the cushioning composite (9) comprises an air gap (G) and one or more nonwoven layers, preferably being positioned at an apex position (Ap, AP') of the front and/or rear waist belts (2, 3) that circumscribes the opening (Ow).

4. An absorbent article according to any of the preceding Claims wherein the cushioning composite (9) is formed by one or both of the inner and outer nonwoven layers (6, 5).

5. An absorbent article according to any of the preceding Claims wherein the outer nonwoven layer (5) is wider than the inner nonwoven layer (6) and is folded over the inner nonwoven layer (5) at a waist edge (10, 10') and wherein an air gap (G) is formed between the outer and inner nonwoven layers (6, 5) at said waist edge (10, 10').

6. An absorbent article according to Claim 5 wherein an apex position (Ap, Ap') of the front and/or rear waist belts (2, 3) that circumscribes the opening (Ow) comprises the waist edge (10, 10'), preferably wherein the waist edge (10, 10') corresponds to an apex of the front and/or rear waist belts (2, 3) that circumscribes the opening (Ow).

7. An absorbent article according to any of the preceding Claims wherein a separation distance (ds) separates the two nonwoven layers at the bubble-shaped waist rim, and the outer nonwoven layer (5) and inner nonwoven layer (6) are separated from each other at one or more portions other than the substantially bubble-shaped waist rim by a distance (d), and wherein the separation distance (ds) is greater than the distance (d), preferably wherein the separation distance (ds) is at least 1.2d, even more preferably the separation distance (ds) is from 1.5d to 20d.

8. An absorbent article according to any of the preceding Claims-9 wherein one of the inner nonwoven layer (6) or outer nonwoven layer (5) has a basis weight that is greater than the other of the outer nonwoven layer (5) or the inner nonwoven layer (6), preferably wherein the outer nonwoven layer (5) has a basis weight that is greater than that of the inner nonwoven layer (6), even more preferably wherein the outer nonwoven layer (5) has a basis weight that is at least 5gsm greater than the inner nonwoven layer (6).

9. An absorbent article according to any of the preceding Claims wherein the outer nonwoven layer comprises, preferably consists of, a nonwoven selected from the group consisting of a spunbond nonwoven layer, a carded nonwoven layer, and combinations thereof, more preferably wherein the outer nonwoven layer is a carded airthrough-bonded nonwoven layer or a spunbond nonwoven layer.

10. An absorbent article according to any of the preceding Claims wherein the inner nonwoven layer (6) comprises at least one spunbond nonwoven layer and at least one meltblown nonwoven layer, preferably wherein the inner nonwoven layer (6) comprises, preferably consists of, a spunbond-meltblown-spunbond nonwoven layer.

11. An absorbent article according to any of the preceding Claims wherein the cushioning composite (9) has a height (H, H') extending substantially parallel to a longitudinal axis (y), and the front and rear waist belts (2, 3) have a front width (wf) and a rear width (wr) respectively each extending substantially parallel to the longitudinal axis (y), and wherein said height (H, H') is less than 50% of said front width (wf) and/or rear width (wr), preferably wherein said height (H. H') is from 5% to 30% of said front width (wf) and/or rear width (wr).

12. An absorbent article according to any of the preceding Claims wherein the cushioning composite (9) has a height (H) extending substantially parallel to a longitudinal axis (y), and wherein each one of the pair of side seams (8, 8') has a height (h) extending substantially parallel to a longitudinal axis (y), and wherein the height (H) of the cushioning composite (9) is less than 40% of the height (h) of the seam (8, 8'), preferably from 5% to 35% of the height (h) of the seam (8. 8').

13. An absorbent article according to any of the preceding Claims wherein the cushioning composite (9) is free of further nonwoven layers beyond the inner and outer nonwoven layers (6, 5).

14. An absorbent article according to any of the preceding Claims wherein the cushioning composite is positioned on both the skin-facing surface and the garment-facing surface of the front and/or rear belts (2, 3).

15. An absorbent article according to any of the preceding Claims wherein the inner nonwoven layer (6) is folded such to comprise an inner fold having a length that substantially corresponds to the height (H, H') of the cushioning composite (9), preferably wherein no elastic strand(s) are sandwiched between adjacent surfaces of the folded nonwoven (6), and preferably an air gap (G) being comprised between an inner surface of the folded inner nonwoven (6) and a garment-facing surface of the outer nonwoven layer (5); and wherein the outer nonwoven layer (5) is folded over the inner nonwoven layer (6) in the same direction of the inner fold of the inner nonwoven layer (6) so that a folded portion of said outer nonwoven layer (5) extends along the length of the inner fold, and an end position of the folded portion of the outer nonwoven layer (5) being joined to the skin-facing surface of the inner nonwoven layer (6) and/or insert (4) at a bond location (11).
